# EUROPEAN PATENT SPECIFICATION

(11) **EP 1 378 212 B1**
(45) Date of publication and mention of the grant of the patent: **30.09.2009**
(21) Application number: 03253775.5
(22) Date of filing: 14.06.2003
(51) Int. Cl.: A61F 2/82

(54) **Stent with radiopaque markers incorporated thereon**
Stent mit integrierten radiopaquen Oberflächenmarkierungen
Stent avec marqueurs radiopaques incorporés sur leur surface

(30) Priority: 27.06.2002 US 183071
(43) Date of publication of application: 07.01.2004
(73) Proprietor: Cordis Corporation, Miami Lakes, Florida 33014 (US)
(72) Inventor: Burgermeister, Robert, Bridgewater, NJ 08807 (US); Majercak, David C., Stewartsville, NJ 08886 (US)
(74) Representative: Belcher, Simon James

(56) References cited:
- EP-A- 0 824 900
- EP-A- 0 938 878
- WO-A-98/48733
- US-B1- 6 312 459

## Description

The invention relates to a stent which has a plurality of struts, with portions which have increased size to increase radiopacity.

A stent is commonly used as a tubular structure left inside the lumen of a duct to relieve an obstruction. Commonly, stents are inserted into the lumen in a non-expanded form and are then expanded autonomously (or with the aid of a second device) *in situ.* A typical method of expansion occurs through the use of a catheter mounted angioplasty balloon, which is inflated within the stenosed vessel or body passageway, in order to shear and disrupt the obstructions associated with the wall components of the vessel and to obtain an enlarged lumen.

In the absence of a stent, restenosis may occur as a result of elastic recoil of the stenotic lesion. Although a number of stent designs have been reported, these designs have suffered from a number of limitations. These include restrictions on the dimension of the stent.

Other stents are described as longitudinally flexible but consist of a plurality of cylindrical elements connected together. This design has at least one important disadvantage, for example, according to this design, protruding edges occur when the stent is flexed around a curve raising the possibility of inadvertent retention of the stent on plaque deposited on arterial walls. This may cause the stent to form emboli or move out of position and further cause damage to the interior lining of healthy vessels.

Thus, stents are known in the art. Such stents may be expanded during or just after balloon angioplasty. As a general rule, the manufacture of a stent will need to compromise axial flexibility in order to permit expansion and provide overall structural integrity. -

Prior stents have had a first end and a second end with an intermediate section between the two ends. The stent further has a longitudinal axis and comprises a plurality of longitudinally disposed bands, wherein each band defines a generally continuous wave along a line segment parallel to the longitudinal axis. A plurality of links maintains the bands in a tubular structure. In a further embodiment of the invention, each longitudinally disposed band of the stent is connected, at a plurality of periodic locations, by a short circumferential link to an adjacent band. The wave associated with each of the bands has approximately the same fundamental spatial frequency in the intermediate section, and the bands are so disposed that the waves associated with them are spatially aligned so as to be generally in phase with one another. The spatial aligned bands are connected, at a plurality of periodic locations, by a short circumferential link to an adjacent band.

In particular, at each one of a first group of common axial positions, there is a circumferential link between each of a first set of adjacent pairs of bands.

At each one of a second group of common axial positions, there is a circumferential link between each of a second set of adjacent rows of bands, wherein, along the longitudinal axis, a common axial position occurs alternately in the first group and in the second group, and the first and second sets are selected so that a given band is linked to a neighbouring band at only one of the first and second groups of common axial positions.

Furthermore, this stent can be modified to provide for bifurcated access, whereas the stent itself is uniform throughout. If the manufacturer designs such a stent to have an large enough opening, then it is possible to place the stent such that a pair of stents can be placed one through the other. In this fashion, the stents are capable of being placed at a bifurcation, without any welding or any special attachments. An interlocking mechanism can be incorporated into the stent design to cause the stent to interlock at the desired position during assembly of the device.

Further, a metallic stent has been designed which contains a repeating closed loop feature. The stent is designed such that the closed loop does not change dimensions during expansion. The composite stent is created by filling the area enclosed by the loops with a material that enhances clinical performance of the stent. The material may be a ceramic or a polymer, and may be permanent or absorbable, porous or nonporous and may contain one or more of the following: a therapeutic agent, a radio-opaque dye, a radioactive material, or a material capable of releasing a therapeutic agent, such as rapamycin, cladribine, heparin, nitrous oxide or any other know drugs, either alone or in combination.

It has been seen, however, that it may be desirable to provide for stents that have both flexibility to navigate a tortuous lesion as well as increased column strength to maintain the rigidity necessary after emplacement into the lumen of the body. The pref erred designs tend to provide the flexibility via undulating longitudinal connectors. The rigidity is generally provided via the mechanism of slotted tubular stents. It is perceived that there may be mechanisms capable of enhancing the characteristics of these types of stents. Such a stent would be both flexible in delivery and rigid upon emplacement.

Furthermore, it is desirable to be able to produce stents in which the cross-sectional profile of either the struts or the connecting members is tapered (or variable) in size. In addition, it may be desirable to modify stents to have non-rectangular cross-sections. In both these cases, different manufacturing methods may aid in the creation of such stents.

In addition, it is sometimes recognized that various stents are not adequately visible under fluoroscopy.

US-6312459 discusses a stent with an increased strut width in regions which have little or no impact on the flexibility or mechanical properties (i.e. strength) of a stent. This increases the radiopacity in those regions.

According to the present invention, there is provided a stent as defined in appended claim 1. In one embodiment a stent has struts with larger metallic areas which locally increase the radiopacity of the stent. This increased area causes that area of increased radiopacity to become more visible when placed on the ends of the stent, so that preferably the stent is more visible under fluoroscopy during placement of the stent.

Embodiments of the invention will now be described by way of example with reference to the accompanying drawings, in which:
Figure 1 is a plan view of a stent useful for understanding the invention;
Figure 2 and 3 are plan views of an alternative stent useful for understanding the invention;
Figure 4 is a plan view of yet another stent useful for understanding the invention;
Figure 5 is a close up of the identified section of Figure 4 taken along lines b-b of Figure 4;
Figure 6 is a schematic of a photoresist pattern formed on the stent in order to perform a method for making the stent;
Fig. 7 is a plan view of yet another alternate stent, useful for understanding the present invention;
Fig. 8 is a plan view of a further alternate stent, useful for understanding the present invention; and
Figs. 9 and 10 are schematics of the theory behind expansion of the stent of Fig. 8;
Figure 11 describes a plan view of a stent incorporating a feature of the current invention; and
Figure 12 is a plan view of an alternate embodiment of the feature of the current invention as seen in Figure 11.

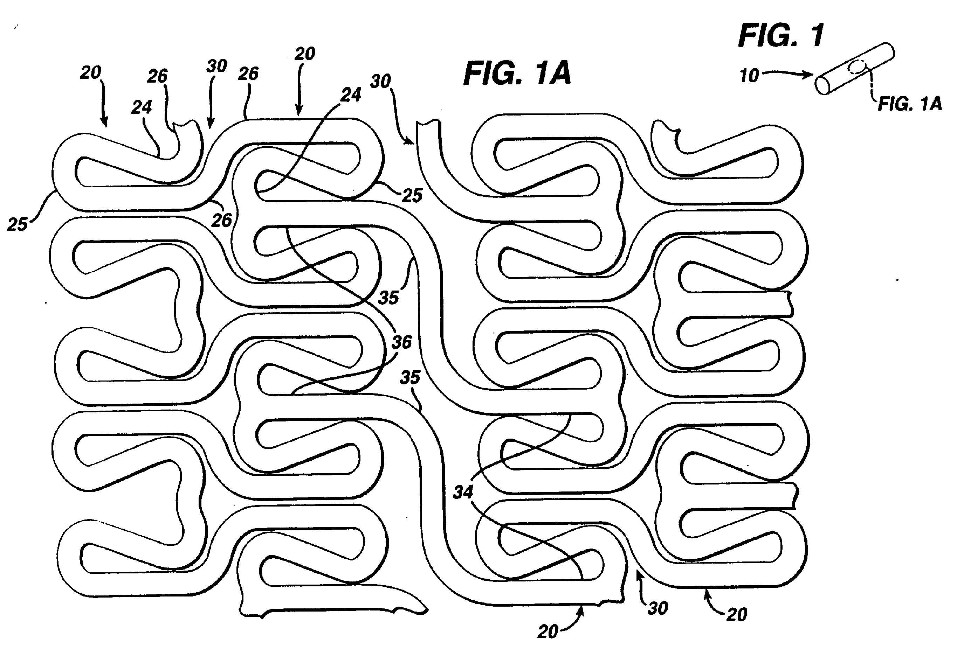

Referring to the drawings, Figure 1 shows a cylindrical stent 10 which has a series of folded strut sections 20 connected by a series of flexible sections 30. The folded strut sections 20 comprise a generally folded strut member 25 having a pair of ends 24, 26. Each of the pair of ends 24, 26 is connected to another folded strut member 25 and also to the end of a flexible member 35. Thus, each end 34, 36 of a flexible member 35 is connected to two ends 24, 26 of a folded strut 25 section member.

Each of the folded struts 25 takes on a generally irregular pattern. On the other hand, each of the flexible sections 35 takes on a generally undulating pattern. The folded strut sections 20 wrap circumferentially around the cylindrical shape of the stent 10. Each flexible section 30 also connects to a folded strut section 20 around the circumference of the stent. It will be noticed that each adjacent flexible section 30 is positioned 180° out of phase with each other.

The longitudinal lengths of the folded struts 20 are short enough to give a smooth profile when the stent is bent. The folded strut 20 allows for a large diametrical expansion range upon expansion. So, upon expansion, the folded struts 20 expand circumferentially and become hoop-like so that maximum radial strength is achieved. The flexible members 30 placed between the folded struts improve the stent deliver ability in the unexpanded dimension of the stent 10. These flexible members are longitudinally compliant so that foreshortening is minimized upon expansion.

In use, therefore, the stent 10 is placed on a balloon catheter and is snaked through the vasculature to be placed into a lesion site in an artery, typically a coronary artery. Because the flexible sections 30 are so substantially flexible, they are able to navigate tortuous lesions with relative ease. Once in place, the balloon catheter is expanded by conventional means. Upon expansion, the struts 25 in the folded strut sections 20 expand to obtain a hoop-like shape. In addition, these members expand longitudinally, so that any reduction in foreshortening is negated. Of course, upon expansion, the flexible members 35 straighten so that there is further strength achieved by the stent in the straightened and rigid positions.

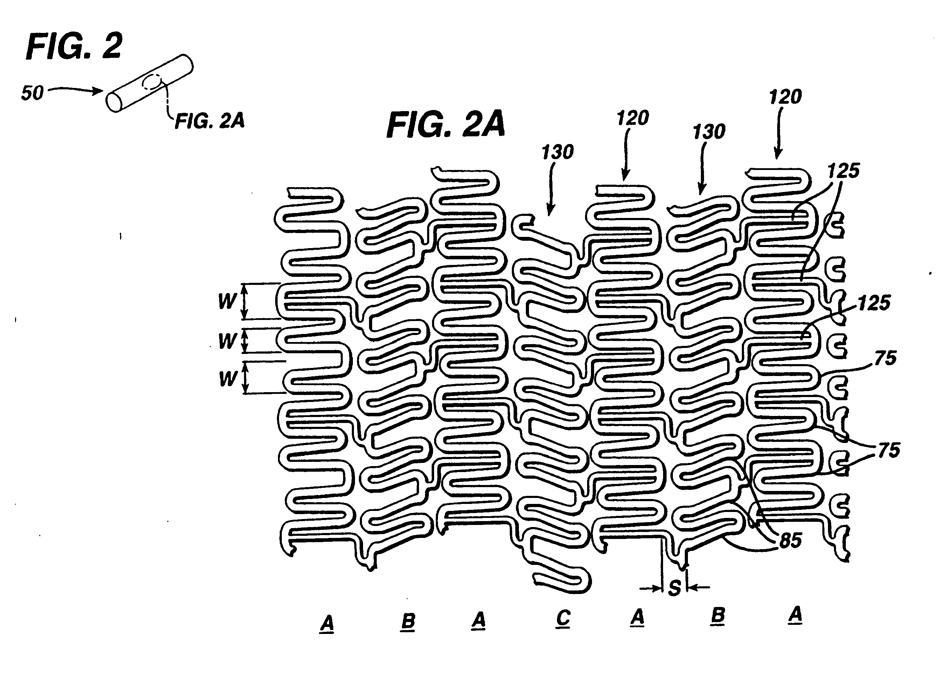

A variation can be seen in the stent 50 of Figures 2 ("angled" version) and 3 ("straight" version). There, the radial strength sections 120 are achieved with generally straight members 115, although these members do not have folded struts. Connection between generally straight members 115 is made by connecting the generally straight members 115 to the more flexible members 125, much like the connection made involving the connecting members of Figure 1.

The members that reduce foreshortening are angled members 130 which are seen to be 180° out of phase with one another. The connection between the flexible members is made at the end of a particular relatively non-flexible member and at the distal end of a particular angled canted member 130. Now, when the columns comprised of relatively rigid members 115 expand, the length of these members 130 shorten. But, the longitudinal lengths of the canted members 130 are placed at an angle compared to the longitudinal axis of the stent 50. So, upon expansion, these canted members 130 actually *lengthen* with respect to the longitudinal axis of the stent 50. The net result is that no foreshortening occurs upon expansion of stent 50.

The canted members 130 are angled in order to both: increase flexibility; and to provide additional resistance on the balloon surface. This arrangement helps prevent what is known as "dogboning" or exposure of leading edge of any of the strut members 75 contained at either end of the stent 50. In addition, this configuration also prevents slippage of the stent along the balloon surface. The canted members 130 are canted in opposite phase (i.e., with a phase change of 180°) to one another, in order to negate any torsional effects on the struts 75, 85 along the length of the stent. These particular members can be crimped to a lower profile than the more rigid members, in order to ensure increased retention of the stent on the surface of a balloon catheter. Further, the configuration described herein has a uniquely folded configuration reducing any risk of "flaring" of the edges of struts 75, 85 during the traversal of the lumen.

It is to be noticed that the longitudinal position (the "order") of the columns can be changed if one desires a smaller initial profile. That is, if one desires that the profile be smaller, it is possible to remove the more rigid sections 120 (or portions thereof) and replace them with the generally canted sections 130.

It is also to be noticed that the wave amplitudes of the struts in a particular column are not kept constant. The wave amplitudes, defined herein as "W", can be lengthened where permitted by the geometry. For instance, notice the space S created between one set of strut members A and a second set of strut members B. This particular configuration allows an increased expansion range around the unexpanded circumference of the stent, while maintaining an appropriate expansion area associated with the metallic struts placed around of the circumference of the stent. Such optimization of the strut surface area is important to ensure adequate coverage of the lesion upon expansion of the stent.

The stent 50 is expanded in much the same way as the stent 10 of Figure 1. When expansion occurs via the balloon catheter, the canted members 130 tend to lengthen and prevent foreshortening of the stent 50; the relatively rigid members 120 tend to shorten in the longitudinal direction, but in so doing provide a greater rigidity for the fully expanded stent. It is to be understood however, that in the expansion of both stents 10, 50 the ability to flexibly navigate the vasculature is enhanced from configuration of either stent 10, 50, as the case may be. All the while, the likelihood of stent foreshortening upon expansion is greatly reduced.

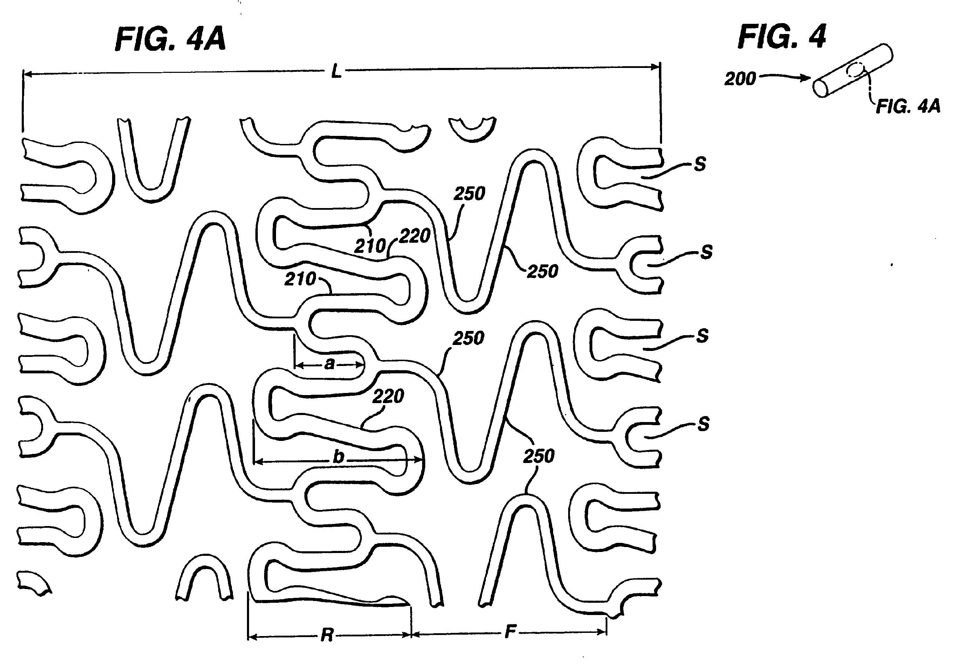

As can be seen in Figure 4; one can also provide for a stent 175 that does not contain canted sections. Yet, the stent 175 expands with decreased foreshortening along its length due to the unique geometry of the stent 175. Here, the stent struts 180, 190 provide for a relatively constant length along the longitudinal axis. (In other words, the longitudinal dimension of the struts 180, 190 in combination remains relatively constant, whether in the expanded or unexpanded condition.) In this fashion, upon expansion, the stent 175 maintains a generally constant length in any of its expanded, unexpanded or partially expanded conditions.

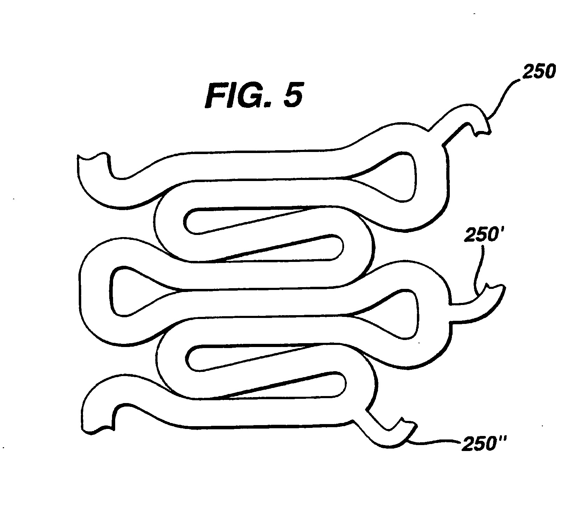

Figures 4 and 5 show the design of a similar stent 200. Here, the connector 250 is shaped like an "N", much after the same fashion of "N"-shaped connectors found commercially in the stent sold by Cordis Corporation under the trade mark Bx Velocity, some details of which are disclosed in US-6190403, and the European patent application which claims priority from US patent application no 09/636071.

In the stent 200, the relatively rigid sections R contain unequal struts 210, 220 of lengths a, b, as can best be seen in Figure 4. Moreover, as can be seen in Figure 5, this strut pattern is formed so that the attachment points a at the end of the flexible connectors 250 can be located at any point along the struts 210, 220 rigid section. In this fashion, when the stent is expanded, the relatively more rigid section R "holds" the connector 250 along the surface of the lesion, so that tenacity of the stent, and its concomitant support are both maintained to a high degree at the situs of the lesion. Yet, in the unexpanded configuration, the "N"-shaped flexible connectors 250 are able to guide the stent 200 around the curvature of generally any tortuous vessel, including tortuous coronary arteries.

As can be seen from Figures 4 and 5, the stent 200 is also capable of reducing foreshortening along its entire length. This stent contains relatively rigid sections R and relatively flexible sections F containing connectors 250. (The flexible sections F are in the form of undulating longitudinal connectors 250.) The relatively rigid sections R generally contain a slotted form, created with struts 210, 220 around a slot S. The relatively rigid sections R contain these interlaced struts 210, 220, which are of varying longitudinal dimensional length.

As can be seen from the figures, in some radial positions, the struts 210 are made longer. In other radial positions, the struts 220 are made shorter. However, the shorter struts 220 are of a constant length b in the longitudinal dimension, and in the fashion in which they connect to the relatively flexible connectors 250. Also, as described above, the relatively more rigid sections R maintain the relatively more flexible sections F at a generally constant longitudinal length due to the friction maintained by the relatively more rigid sections R on a balloon portion of an angioplasty type balloon catheter. Accordingly, upon expansion, the constant length b, in conjunction with the generally constant length of the relatively flexible connector 250, causes the stent 200 to maintain a relatively constant longitudinal dimension L in any diameter to which it is expanded. As can be appreciated, the maintenance of a constant length is desirable from the perspective of secure, repeatable placement of the stent within the vasculature.

Continuing to describe the stent 200 of Figures 4 and 5, the flexible sections F operate with the behaviour of the flexible connectors 250 acting in the fashion of "N"-shaped flexible connectors of similar type. That is, the flexibility of the stent 200 is focussed in this area F so that one is able to traverse tighter lesions using such a configuration. The relatively stronger sections R are capable of expansion to a stronger plastically deformed dimension, so that in this fashion the stent 200 is capable of supporting the arterial wall. Even though the longitudinal dimensions of the struts 210, 220 in the relatively stronger sections R are of unequal length, such a configuration does not diminish radial support in the expanded condition. Accordingly, it can be appreciated that a stent of this shape will adequately support the arterial walls at the lesion site, while maintaining radial flexibility, and longitudinal length.

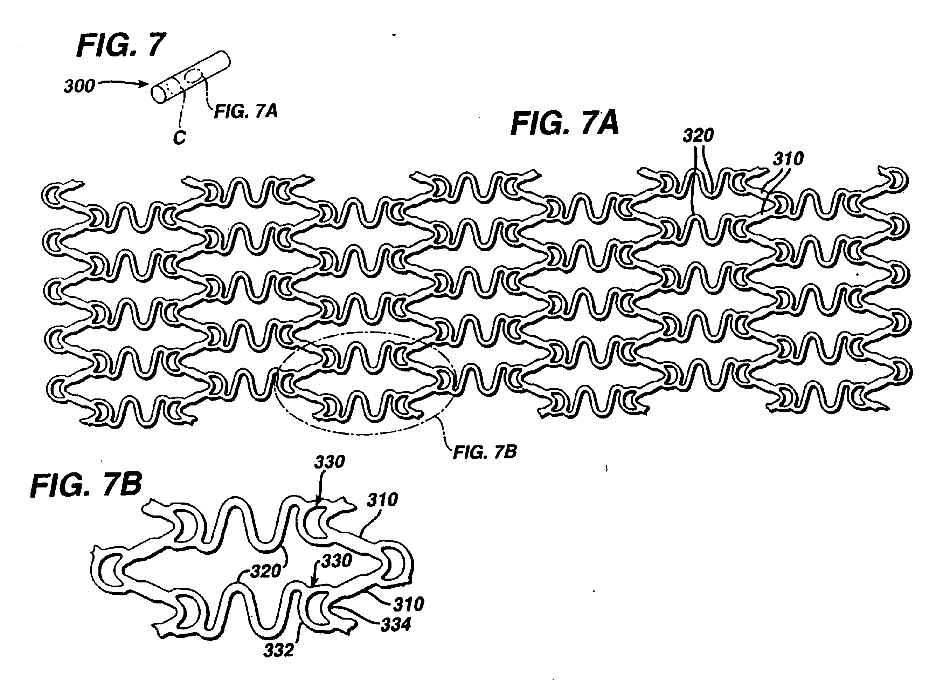

In Figure 7, there is contained a stent 300 much like the stent sold by Cordis Corporation under the trade mark Bx Velocity. In Figure 7 there is contained on the stent 300 generally flexible connector members 310 connected to generally rigid radial strut members 320. The connector members 320 are generally formed in the shape of the letter "N", and the struts 310 are generally slots formed in a radial fashion around the circumference of the stent. The connection made between the flexible connectors 320 and the radial strut members 310 is formed from a living hinge 330. This living hinge 330 contains outer radial arc 332 and an inner radial arc 334. In the expanded configuration, the radial arcs 332, 334 move away one from the other, so that the overall length of the living hinge 330 actually increases upon expansion.

Known conventional means, such as angioplasty balloons, or the balloon on a stent delivery system expands the stent 300 of the present invention. Upon expansion, there are provided a number of benefits by the stent 300 of the present invention. First, as explained above, there is reduced foreshortening of the stent 300, since the outer radial arc 332 in fact does not foreshorten. Since it lengthens slightly, the overall length of the stent 300 is maintained to its general nominal length. There is also provided increased radial strength since the radial arcs 332, 334 at their connection between the flexible and radial struts 320, 310, (both inner and outer radial arcs 334, 332) combine to give superior strength in the section of the arcs; the radial strut 310 provides for optimal strength in the radial direction since it is parallel to the loading direction of the stent 300, thereby creating a "hoop" a circumference C of the stent. Also, because the radial arcs are able to accept greater forces, there is reduced strain for the equivalent strength designed for a stents. In all, the stent 300 provides for at least equivalent radial strength, less foreshortening and reduced strain when compared to current stents.

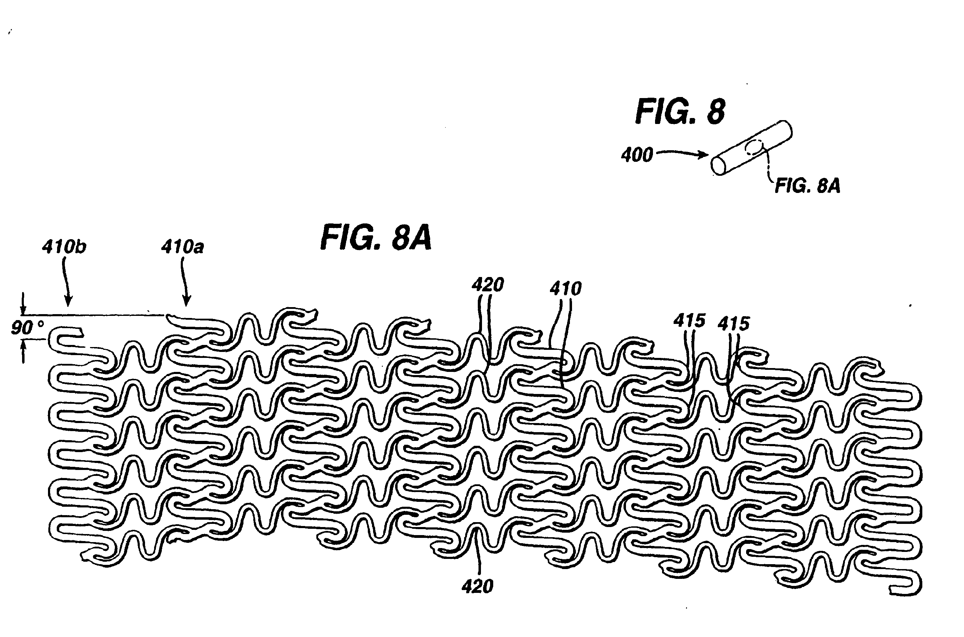

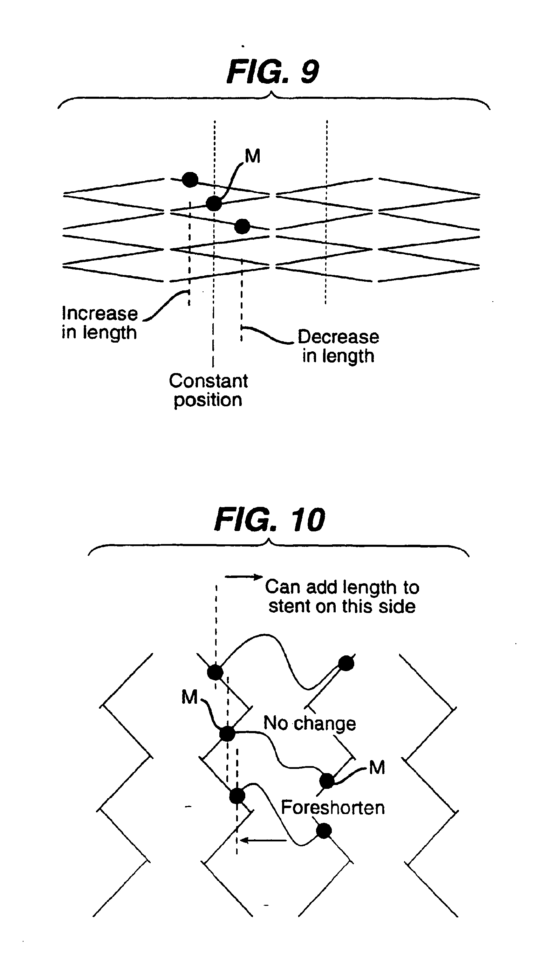

As can be seen from figures 8, 9 and 10, there is provided the stent 400. Again, the stent 400 provides for generally stronger radial sections R comprising radial struts 410, which are generally slotted in alternating fashion around the circumference of the stent.
The flexible connector members 420 are similar to the flexible connector members as seen in Figure 7, and also to the flexible connector members of the Bx Velocity stent. However, these flexible connector members 420 are connected to the radial struts generally somewhere near the midpoint of the radial struts 410. In this fashion, upon expansion the length of the connector members 420 remains independent of the shortening or lengthening of the radial struts 410. In this way, the overall length of the stent is maintained, as seen from the schematics in Figures 9 and 10.

Due to this overall ability to maintain the length of stent 400, the radial struts 410 provide for radial strength only, and do not contribute in one way or another to any foreshortening of the stent. Also, the radial struts 410 are formed from a generally "wavy" pattern. This wavy pattern is useful in helping to reduce the crimp profile of the stent 400 on the balloon. This results from the relative smooth attachment of the radial struts 410 to the flexible connectors 420. Further, having such an arrangement reduces the strain placed on the struts 420 upon expansion. This reduced strain is achieved due to the location of the connection of the struts 420 to the struts 410. Because there is relatively little movement of the struts 420 in the longitudinal direction, there is relatively little strain placed on these struts during expansion. The radial arcs 415 of struts 410 can be ideally placed in a "shifted" configuration so that the stent is easier to crimp on a balloon.

Further, this can be seen from Figure 8, that the radial strut members 410 are attached to the flexible connectors 420 so that the flexible connectors 420 generally proceed along a "spiral" pattern S around the length of the stent 400. The connection points 422 of the flexible connectors 420 are placed in a diagonal fashion on struts 410 so as to enhance flexibility. Generally connectors 422 are located in a midpoint of a strut 410. When the connectors 422 are placed past the midpoint of strut 410 (i.e. farther from the midpoint of strut 410 then from the direction of connector 420), the nominal stent strength should increase upon expansion when compared to the above described stent. This arrangement reduces foreshortening, as described above. Further, the arrangement in no wise affects any torsion on the stent as it is applied to the lumen by the balloon catheter. Friction of the balloon to struts 410 maintains the struts 410 (and their opposite struts 420) in the same general radial position throughout expansion. By reducing any concern of stent torsion, there is also a reduced concern of overall slippage of the balloon. Even though the connector members 420 are not aligned with one another, they are maintained in their respective positions on the balloon surface. Upon expansion, struts 420 lock into place, as the stent 400 is placed, giving an increased strength in the lumen.

From Figures 8 and 9, we see that the midpoint of a connector 420 is important to maintaining length. The greater the distance from connector 420 to the midpoint M, on the side of the connection between struts 410, 420, the greater the potential for shortening of the stent. This creates a need to solve any shortening by other means, absent the solution described herein.

It is to be understood that various modifications to the stent 400 of Figures 8, 9 and 10 are possible. For instance, the connectors 420 can be placed intermittently about the stent 400 circumference, and not at every incidence of a radial strut 410. Also, while the radial struts 410 are generally 90° out of phase between one series of struts 410a and the next 410b, it is foreseeable to place them between 30° and 150° out of place. When so placed, the strut 410 can be "encouraged" to bend in a particular fashion, which may be preferential in the design of a particularly intended stents.

These stents can be manufactured by know conventional means, such as laser etching, electrical discharge machining (EDM), photochemical etching, etc. However, there is also disclosed in the invention herein a novel method of performing photochemical resistance etching of the tube from which the stent is to be made. This novel method allows one to produce a stent with variable geometry in the three dimensions of the strut, that is, along its length, across the circumferential dimension, and along its depth (or radial dimension.) This method starts with a standard photochemical machining process.

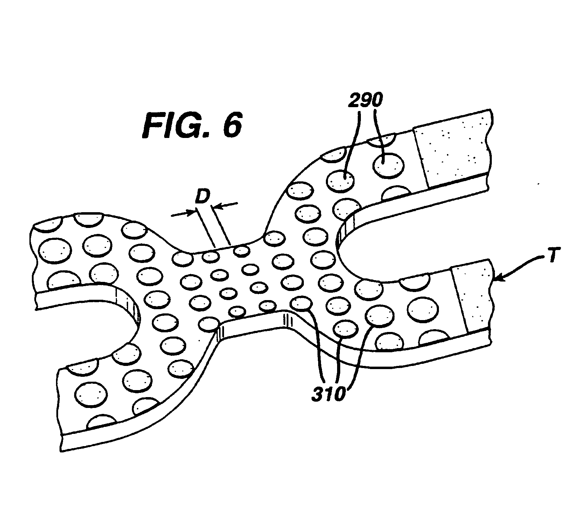

The new process consists of cutting the stent using photochemical etching, cleaning it, and then coating it with a photoresist. The photoresist coating is applied in circular shapes 290, as can be appreciated from Figure 6. These shapes 290 are intentionally figured to be of varying dimension in their radius. Then, a photoresist image is developed on the surface of the cylindrical metallic tube T from which the stent starts.
This photoresist image is developed in a controlled fashion using known means. The development of the photoresist in this fashion allows a controlled variable etching rate at select positions along the cylindrical metallic tube.

As previously stated, the novel photoresist image can be seen in Figure 6. This photoresist image consists of a series of circular regions of photoresist material 310, which are shaped in a variable diameter as desired for manufacture. These photoresist images 310 are configured at variable distances D from one another. As the diameter of the circular photoresist pattern 310 decreases, and its distance from other photoresist patterns 310 increases, the etching rate of that area of the stent increases. Thus, by strategically placing the photoresist patterns 310 on the stent, one can produce any variable dimension in any direction along the stent.

This photoresist pattern 310 variation results in a variation in the metal of the stent removed during the etching process. This process can be used to locally change the geometry of the metallic tube.

In this fashion, one can envision making a stent of variable circumferential width, radial depth or longitudinal length. As such, one can impart varying flexibilities along the stent longitude, as well as varying strengths so that a stent can be configured for emplacement at various locations within the body.

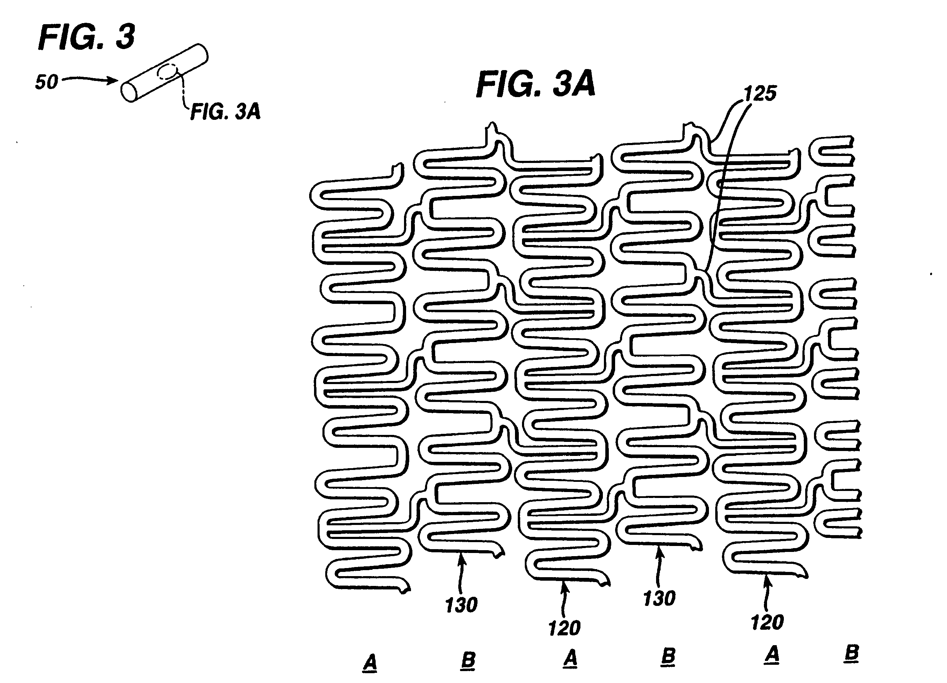

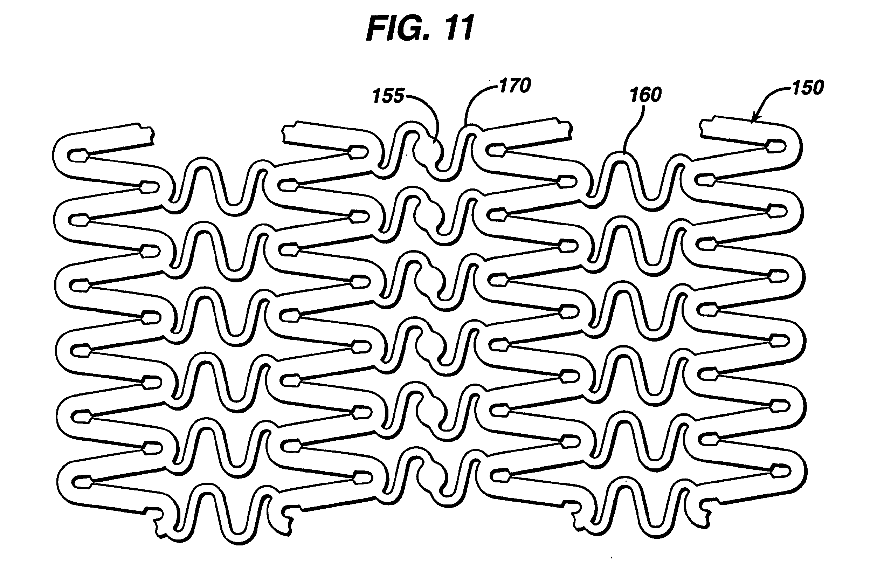

As seen in Figure 11 there is described a stent 150 with an additional radiopaque stent marker 155 incorporated therein according to an embodiment of the present invention. In contrast to other stents with radiopaque markers, these radiopaque markers 155 are formed integral with the stent 150. That is, the flexible connectors of the stents as seen in Figures 1-10 are typically formed in a similar way as those flexible connectors 160 placed at the right and left ends of the stent. However, in the centre of the stent there is incorporated a larger radiopaque dot 155 placed on the flexible connectors 170. This radiopaque dot 155 is formed integral with the device, and so there is no possibility of antigalvinic effect taking place on the stent 150 after implantation. Yet, because there is a larger surface area as contained in the radiopaque dot 155 on flexible connectors 170 of the stent 150, it is realized that this marker is able to be better seen under fluoroscopy. In this vein, the stent 150 is more visible during delivery.

Alternately, the radiopaque dots are placed at both ends of the stents so that both ends of the stent are more visible during delivery. Of course, the stent can be incorporated with any of these such radiopaque dots placed in any fashion, and their placement is merely a suggestion for the current device.

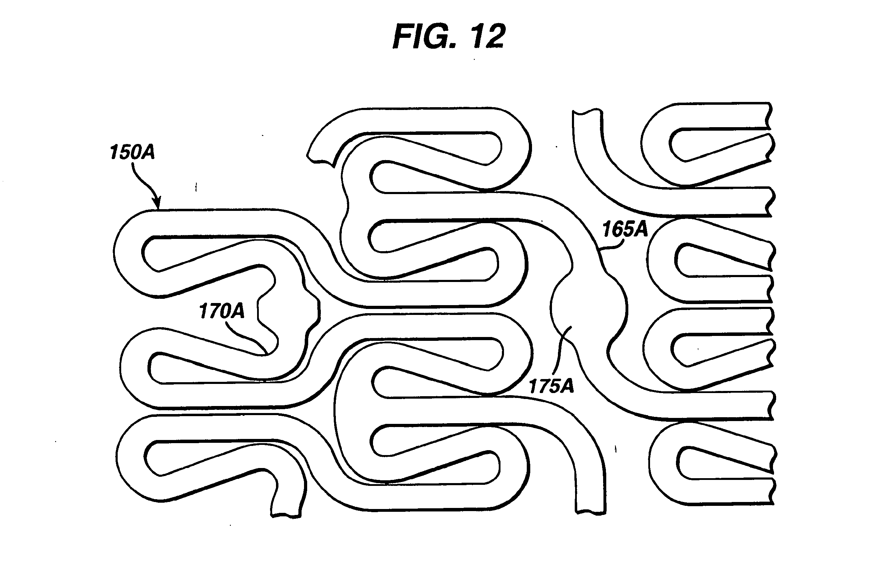

As seen in Figure 12, an alternate device 150A also incorporates these dots 175A.
They can be placed on the flexible portions 165A, and on more rigid portions 170A, as desired by the user.

## Claims

1. A stent comprising:
flexible portions (170, 165A) and more rigid portions (170A) each of the flexible portions (170, 165A) and more rigid portions (170A) comprising a plurality of struts,
wherein at least some of said struts have an area of increased size (155, 175A) so that said area of increased size is more radiopaque under fluoroscopy; **characterised in that** the area of increased size (155, 175A) is placed on a strut in a flexible portion (170, 165A).

2. The stent of claim 1 wherein the area of increased size (155, 175A) is placed on the strut contained at the ends of the stent.

3. The stent of claim 1 or 2 wherein the stent is made from stainless steel.

## Patentansprüche

1. Stent, welcher Folgendes umfasst:
flexible Teilbereiche (170, 165A) und starrere Teilbereiche (170A), wobei die flexiblen Teilbereiche (170, 165A) und die starreren Teilbereich (170A) jeweils eine Vielzahl von Streben umfassen,
wobei zumindest einige der Streben eine Fläche erhöhter Größe (155, 175A) aufweisen, so dass die Fläche erhöhter Größe röntgenstrahlenundurchlässiger unter Fluoroskopie ist; **dadurch gekennzeichnet, dass** die Fläche erhöhter Größe (155, 175A) auf einer Strebe in einem flexiblen Teilbereich (170, 165A) angeordnet ist.

2. Stent nach Anspruch 1, wobei die Fläche erhöhter Größe (155, 175A) auf der Strebe angeordnet ist, welche an den Enden des Stents enthalten ist.

3. Stent nach Anspruch 1 oder 2, wobei der Stent aus nicht-rostendem Stahl gefertigt ist.

## Revendications

1. Stent comprenant des parties flexibles (170, 165A) et des parties plus rigides (170A), chacune desdites parties flexibles (170, 165A) et des parties plus rigides (170A) comprenant une pluralité de montants, dans lequel au moins une partie desdits montants a une superficie de taille supérieure (155, 175A), de sorte que ladite superficie de taille supérieure soit plus radio-opaque sous fluoroscopie ; **caractérisé en ce que** la superficie de taille supérieure (155, 175A) est placée sur un montant dans une partie flexible (170, 165A).

2. Stent selon la revendication 1, dans lequel la superficie de taille supérieure (155, 175A) est placée sur le montant contenu aux extrémités dudit stent.

3. Stent selon les revendications 1 ou 2, dans lequel le stent est réalisé en acier inoxydable.
